# EUROPEAN PATENT SPECIFICATION

(11) **EP 2 499 265 B1**
(45) Date of publication and mention of the grant of the patent: **11.09.2019**
(21) Application number: 10829390.3
(22) Date of filing: 27.07.2010
(51) Int. Cl.: C12Q 3/00, C12M 1/36, C12M 3/00, C12N 5/00, C12M 1/34

(54) **DEVICE AND METHOD FOR CULTURING CELLS**
VORRICHTUNG UND VERFAHREN ZUR ZELLKULTIVIERUNG
DISPOSITIF ET PROCÉDÉ DE CULTURE DE CELLULES

(30) Priority: 13.11.2009 US 272878 P
(43) Date of publication of application: 19.09.2012
(73) Proprietor: The Governing Council of the University of Toronto, Toronto, ON M5S 1A1 (CA)
(72) Inventor: ZANDSTRA, Peter, Toronto Ontario M4R 1Y2 (CA); CSASZAR, Elizabeth, Toronto Ontario M1S 1E9 (CA); KIROUAC, Daniel Christopher, Arlington, Massachusetts 02476 (US); ITO, Caryn, Ottawa Ontario K1S 0P8 (CA)
(74) Representative: Elsy, David
(86) International application number: PCT/CA2010/001180
(87) International publication number: WO 2011/057380

(56) References cited:
- EP-A1- 1 270 718
- EP-A1- 1 816 188
- US-A1- 2002 058 338
- US-A1- 2004 029 264
- JASON E DOWD ET AL: "Optimization and control of perfusion cultures using a viable cell probe and cell specific perfusion rates", CYTOTECHNOLOGY, KLUWER ACADEMIC PUBLISHERS, DO, vol. 42, no. 1, 1 May 2003 (2003-05-01), pages 35-45, XP019236785, ISSN: 1573-0778, DOI: 10.1023/A:1026192228471
- ELIZABETH CSASZAR ET AL: "An automated system for delivery of an unstable transcription factor to hematopoietic stem cell cultures", BIOTECHNOLOGY AND BIOENGINEERING, vol. 103, no. 2, 1 June 2009 (2009-06-01), pages 402-412, XP055094161, ISSN: 0006-3592, DOI: 10.1002/bit.22297
- ELIZABETH CSASZAR ET AL: "Rapid Expansion of Human Hematopoietic Stem Cells by Automated Control of Inhibitory Feedback Signaling", CELL STEM CELL, ELSEVIER, CELL PRESS, AMSTERDAM, NL, vol. 10, no. 2, 6 January 2012 (2012-01-06), pages 218-229, XP028397787, ISSN: 1934-5909, DOI: 10.1016/J.STEM.2012.01.003 [retrieved on 2012-01-10]
- KIROUAC ET AL.: 'Cell-cell interaction networks regulate blood stem and progenitor cell fate' MOLECULAR SYSTEMS BIOLOGY vol. 5, 28 July 2009, pages 1 - 20, XP055093851
- NAKAMIZO ET AL.: 'Human bone marrow-derived mesenchymal stem cells in the treat ment of gliomas' CANCER RESEARCH vol. 65, no. 8, 15 April 2005, pages 3307 - 3318, XP002590133
- KROSL ET AL.: 'In vitro expansion of hematopoietic stem cells by recombinant TAT-HOXB4 protein' NATURE MEDICINE vol. 9, no. 11, November 2003, pages 1428 - 1432, XP003016542
- CSASZAR ELIZABETH ET AL: "Real-time monitoring and control of soluble signaling factors enables enhanced progenitor cell outputs from human cord blood stem cell cultures.", BIOTECHNOLOGY AND BIOENGINEERING JUN 2014, vol. 111, no. 6, June 2014 (2014-06), pages 1258-1264, ISSN: 1097-0290
- CSASZAR ELIZABETH ET AL: "Blood stem cell fate regulation by Delta-1-mediated rewiring of IL-6 paracrine signaling", BLOOD, vol. 123, no. 5, January 2014 (2014-01), pages 650-658, ISSN: 0006-4971(print)

## Description

### Cross-Reference to Related Applications

The present disclosure claims priority from U.S. provisional patent application no. 61/272,878, filed November 13, 2009.

### Technical Field

The present disclosure relates to devices and methods for culturing hematopoietic stem cells and/or progenitor cells.

### Background

Umbilical cord blood (UCB) has been used therapeutically as a source of hematopoietic stem cells (HSCs). Although these cells have several advantages, the limited number of primitive progenitors and long-term repopulating stem cells in an UCB unit may limit its utility. Efforts to expand UCB *in vitro* have included optimizing cytokine levels, co-culturing with stromal cells, selecting the starting cell population, overexpressing target genes, and removing unwanted factors [1-3]. Although relatively significant expansions of total cell and committed progenitor cell numbers have been achieved, the current ability to expand primitive human progenitors and stem cell numbers remains modest, which may be due to a relative lack of understanding of how complex microenvironments can be specifically modified to target the growth of these cells.

The *in vitro* hematopoietic cell culture system is influenced by exogenous factors that are added directly to culture and endogenous factors secreted by the heterogeneous cell populations typically present or emerging in these cultures. Under traditional culture conditions, the cell population begins to lose its stem cell characteristics as the population of mature terminally differentiated cells greatly increases and the concentrations of factors secreted by these mature and progenitor cells subsequently increases. It may be that inhibitory factors drive down the self-renewal of the stem cell population.

The in vitro heterogeneous hematopoietic stem cell culture system is a complex system in which the secretion of endogenous regulatory factors is a dynamic function of the changing cell population. Even in examples where the culture begins in an essentially homogeneous state, the culture system becomes heterogeneous due to emergence of different cell types over time. Although some efforts have been made to inhibit specific negative factors (or add specific positive factors), these approaches have been met with limited success, possibly because they do not take into account the complexity or the dynamic nature of the system. It has been shown that a very large number of factors are secreted by the heterogeneous cell population, and it may not be possible or feasible to add or remove all of these factors in a combined optimized manner. Moreover, the dynamics of the system are such that concentrations of negative secreted factors are continuously changing over time, which may not be easily neutralized by inhibiting individual factors. EP 1270718 describes an automated cell culturing device which computes cell image data using a camera.
EP 1816188 describes a device which measures nutrients and cell density to calculate a desired intracellular reaction rate.
Dowd J.E. et al (Cytotechnology (2003) 42(1) 35 to 42) describes a device which computes cell viability using directive spectroscopy.
Csaszar E et al (Biotech and Bioeng, (2009) 103(2) 402 to 412) describes a device which delivers TAT HoxB4 protein determined by mathematical modelling.

### Summary

In some example aspects there is provided the use of a system for culturing hematopoietic stem cells and/or progenitor cells as defined in claim 1.

The concentration of the at least one marker component is measured continuously or intermittently; and the culture media delivery rate may be determined based on the measured concentration or density of the at least one marker component.

In some example aspects, there is provided a method of culturing cells as defined in claim 10.

The culture media delivery rate may be determined in order to maintain the measured concentration or density of the at least one marker component below a predetermined threshold value.

The disclosed devices and methods may be useful for culturing stem cells and/or progenitor cells, among other cell types.

### Brief Description of the Drawings

Reference will now be made to the drawings, which show by way of example embodiments of the present disclosure, and in which:
FIG. 1 is a schematic illustration of an example device for culturing cells;
FIG. 2 shows charts illustrating simulated cell output, using an example mathematical model;
FIG. 3 shows charts illustrating simulated cell growth, using an example mathematical model;
FIG. 4 shows charts illustrating cell output using an example device for culturing cells compared to conventional cell culture;
FIG. 5 shows charts illustrating cell growth at different culture media dilution rates using an example device for culturing cells;
FIG. 6 shows charts illustrating cell growth using an example device for culturing cells compared to conventional cell culture using an inhibitor;
FIG. 7 shows charts illustrating cell output using an example device for culturing cells compared to conventional cell culture;
FIG. 8 shows charts illustrating simulated results comparing output using an example device for culturing cells compared to conventional cell culture with perfusion;
FIG. 9 shows a chart comparing an example mathematical model with example experimental results for certain cell outputs at day 8;
FIG. 10 shows a chart comparing an example mathematical model with example experimental results for certain cell outputs at day 12;
FIG. 11 shows a chart illustrating simulated secreted factor profiles from conventionally grown cells, using an example model;
FIG. 12 shows a chart illustrating simulated secreted factor profiles, using an example model, from cells grown using an example method of the present disclosure; and
FIGS. 13A-13D show tables listing example stimulators and inhibitors.

### Detailed Description

Existing conventional bioreactors typically are not specifically designed for expansion of stem cell populations. For example, such conventional bioreactors typically do not include tight control of components (e.g., concentration of secreted factors and other proteins) that may be important for stem cell (e.g., hematopoietic stem cells) growth. Existing conventional fedbatch systems and perfusion systems typically do not provide specifically for expansion of stem cell populations.

Typically, conventional fedbatch bioreactors have been used to monitor and control levels of nutrients and/or waste (e.g., glucose and/or other cell metabolites). However, such traditional metabolites may not be limiting factors for certain cells, such as stem cells (e.g., hematopoietic stem cells). Thus, conventional bioreactors typically do not have any mechanism or strategy for monitoring and/or controlling for components (e.g., concentration of secreted factors and other proteins) that are important for stem cell growth. As such, culturing stem cells using such conventional systems may not achieve a desired stem cell population expansion.

In the present disclosure, the accumulation of one or more components (e.g., growth inhibiting factors) that impact cell growth, such as stem cell growth, is taken into account. For example, a fedbatch system that includes monitoring (e.g., on-line or off-line monitoring) and control (e.g., through continuous or intermittent dilution) of
such component(s) (e.g., inhibiting factors) may be used for control and growth of cell populations, such as stem cell populations.

The present disclosure describes examples of devices for culturing cells. The example devices may be useful for propagating human blood stem and progenitor cells, or other stem and/or progenitor cell populations. In the device, accumulation of inhibiting factor(s) of cell growth (e.g., endogenously produced negative regulator(s)) may be controlled by measuring their concentration (e.g., directly measured or indirectly measured via surrogates) and by using this measurement to control the rate of media supplementation or negative component (e.g., cell(s) or inhibiting factor(s)) removal. Such monitoring of marker components (e.g., the factor(s) of interest or surrogate(s) for such factor(s)) may allow for feedback control. The control strategy for the device may be based on a mathematical model and/or software program that predicts endogenous factor concentrations and regulates bioprocess parameters.

In some example embodiments, the device includes a cell vessel for culturing cells. The cell vessel may have one or more inlets for introducing culture media, as well as any other suitable agents or components. The culture media may be delivered via a controllable delivery mechanism, such as a pump or a valve connected to the inlet(s). The culture media may be continuously or substantially continuously (e.g., periodically or regularly, such as on an hourly basis, or intermittently) delivered, which results in continuous or intermittent dilution of component (e.g., including biological factors) within the culture vessel. The delivery mechanism may be controllable, for example by coupling to a processor or other controller device, to control the rate of delivery of the culture media and/or other agent. In some examples, the delivery mechanism may itself have a processor that may be programmed to control the rate of delivery. In some examples, the delivery rate is controlled according to a mathematical model of cell culture behavior. In some examples, the delivery rate is controlled based on feedback information derived from continuous, periodic or intermittent monitoring of media and/or cells from the cell vessel.

In example systems-level molecular profiling studies, sets of positive and negative factors present in UCB conditioned media were determined and experimental validation showed that the identified factors did have a positive or negative impact of functional culture outputs, as was predicted [4], [10]. Given the complexity and
dynamic nature of typical stem cell (e.g., hematopoietic cell) cultures, the effect of adding a single factor or small combinations of factors is typically fairly minimal to the overall system. A more global regulation approach may allow for improvements in culture outputs, over what has been achieved with single factor addition or inhibition. By diluting the culture volume over time, all factors (e.g., including endogenously secreted inhibiting factors) will decrease in concentration and this may serve to counteract the rising levels of inhibitory factors. The effects of any stimulatory factors may be augmented, for example by adding any such factors with the delivered culture media.

The device may include a controllable or automated delivery mechanism to allow for the continuous, periodic or intermittent delivery of culture media, which may include factors (e.g., unstable proteins), to cell culture (e.g., hematopoietic stem cell culture). For example an automated system as described in [5] may be suitable. Using this device, a fed-batch media delivery strategy may be implemented, in which fresh culture media may be added to the cell culture continuously or substantially continuously over time, thereby increasing the culture volume at a predetermined rate or rates. The rate(s) of dilution may be controlled by a user, or may be based on a predictive or theoretical model, or may be controlled by a controller device (e.g., based on feedback information), and may be a constant rate or a variable rate. Previous mathematical simulations on the *in vitro* UCB culture system has provided validated temporal data on the rate of cell expansion and the rate of endogenous factor secretion [4]. Thus, the algorithm for the dilution of the cell culture media may be based on these known rates in order to specifically maintain a constant level of one or more selected marker components (e.g., factor(s) and/or surrogate(s)) as cell culture progresses.

A general dilution approach may eliminate or reduce the need to precisely control the addition or removal (or inhibition) of large combinations of factors. The fed-batch delivery strategy globally decreases the concentration of all endogenously secreted factors present in culture and the continuous or substantially continuous nature of the approach may take into account dynamically changing culture conditions. By continuously or substantially continuously diluting the culture media, for example at a determined (e.g., relatively optimal) rate, the inhibitory effect felt by
the cell (e.g., stem cell) population as a result of paracrine and/or autocrine signaling loops may be decreased. As a result, it may not be necessary to specifically account for each factor individually in culture. By controlling the rate of media dilution based on the dynamics of cell culture (e.g., in terms of cell expansion and/or factor secretion) the overall culture system may be controlled, for example by maintaining at least one or more inhibitory secreted factors below a predetermined threshold level.

The disclosed devices and methods may allow for a variable flow rate (e.g., exponential or feedback-based) which may allow the dynamic nature of the cell culture to be taken into account. The device may also provide global control over the culture with relatively minimal manipulations, compared to conventional bioreactors. The device may avoid the need to select for cells, or to retain cells that are cultured in a media perfusion configuration. The device may allow for improved expansion of both stem cells and progenitor cells. The delivery rate of culture media may be predicted (e.g., using a predictive model) and may be set based on measured levels of one or more representative factors or marker components (e.g., transforming growth factor (TGF β)) in the cell vessel, for example such that the levels of these inhibitory factor(s) are maintained below a threshold value. Such representative factors or marker components may be considered "sentinel" factors, in that control of such factors may be adequate to control the global cell vessel environment, however the intention may not be to control such factors specifically or solely, but rather as a way to control for other unmonitored factors in the global cell vessel environment.

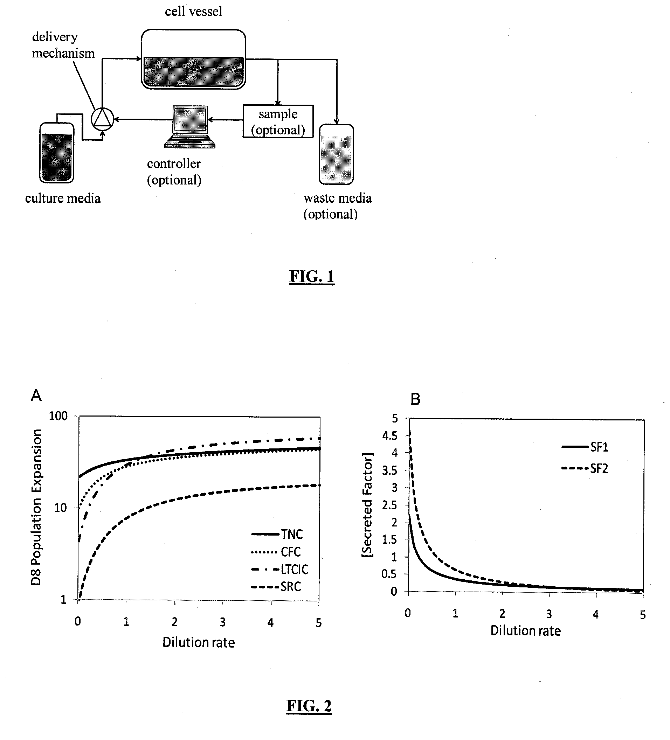

Reference is now made to FIG. 1, showing an example embodiment of a device for culturing cells. In the example shown, the device includes a cell vessel, which may be a sterile, closed bioreactor, such as but not limited to a flask, a culture bag (e.g., made of Teflon or other suitable plastic), an array of microwells or a stirred tank. The device setup may be similar to a fed-batch bioreactor setup. In the example shown, the cell vessel includes both an inlet and an outlet (both of which may be sterile), although in other examples, the cell vessel may include only an inlet or may have additional ports. Fresh culture media is delivered into inlet of the cell vessel, for example from a culture media reservoir, via a delivery mechanism, such as a pump or a valve. The pump is controlled (e.g., by a processor or other controller device) such that the culture media is delivered at a controlled rate. Where the cell vessel includes
an outlet, spent or waste media may be removed from the outlet, for example for perfusion-type feeding or for continuous or semi-continuous compositional analysis. The delivery rate of the culture media is regulated by the controlled delivery mechanism which may regulate flow rate, for example at a constant value, along a fixed trajectory, as a function of endogenous protein concentrations or cell population densities, or according to any other suitable regulation strategy (e.g., based on a model or based on feedback).

In some examples, the device includes a controller device (in this example, shown as a computer) configured for on-line (i.e., real-time) or off-line (i.e., not real-time) continuous or periodic sampling of the cell vessel, for measurement of one or more marker components (e.g., secreted factor levels) which may be used as a feedback control mechanism for delivery of the culture media. In some examples, the marker component may be a surrogate for a secreted factor, where the surrogate is indicative of the level of the secreted factor, such as in cases where the secreted factor itself is hard to detect directly. Although the controller device is shown as a computer, other controller devices may be suitable, including, for example, a processor, an embedded computer chip, or a server. Although the controller device is shown as a separate component from the delivery mechanism, in some examples the delivery mechanism itself may include the controller device (e.g., as a processor or microchip embedded in the delivery mechanism).

This feedback approach, in some examples with real-time continuous or periodic sampling to monitor factor concentration and adjust the culture system correspondingly in real-time, may allow for tighter control of the system and a greater ability to deal with sample to sample variability. The device may be set to dilute culture media in order to maintain one or more marker components (e.g., specific endogenous secreted molecules) below, at, or above a predetermined threshold value. The threshold value may be predetermined based on predictive model(s) and/or experimental data. For example, a technique using a sandwich ELISA-based nanotechnology can be adapted to detect the presence of TGFβ, or other secreted factors, at the picomolar level, with a detection time of less than 1h [9]. This biosensor approach may allow for the detection of TGFβ levels, which could then be
fed back to the controller device or the delivery mechanism to adjust the culture media delivery rate accordingly.

The fresh media delivery rate (and in some examples, the spent media removal rate, where applicable) may be constant or variable and may be a function of the concentration of one or more marker components, such as one or more endogenously produced negative regulators. A variable delivery rate may be set, for example, as a function of the total culture volume in the cell vessel, a preset trajectory, or regulated by feedback control to maintain specific marker component(s) (e.g., one or more endogenous secreted molecules or cell sub-populations) at, above or below a predetermined threshold value. Endogenous secreted molecules used for the marker component may include, for example, ADIPOQ, CCL2, CCL3, CCL4, CCL5, CXCL7, CXCL8, CXCL1O, EGF, PDGF, TGFB1, TGFB2, TNFSF9, or VEGF. Cell populations used for the marker component may include, for example, cells that express CD14, CD15, CD33, CD41, CD235a, CD133, CD34, CD38, CD71, Rho123. Other inhibitory factors that may be monitored as a marker component may include, for example, monocyte-derived inhibitory factors (e.g., CCL3, CCL4, CXCL10, TGFB2 and TNFSF9, as described above)

Compositional analysis for feedback control may include, for example, measuring the concentration of a secreted marker molecule or measuring the density or number of cells expressing a marker or markers, or a surrogate marker of a specific cell type or molecule. In some examples, the cultured cell population may be subjected to continuous or substantially continuous cell sub-population selection for one or a combination of certain phenotypes during the culture. In some examples, the device may include one or more sensors (e.g., a bead-based barcoding sensor, as described in Klostranec et al. [9]) for monitoring (e.g., on-line) of one or more marker components.

In some examples, the composition (e.g., cytokine composition) of the fresh culture media may be varied in accordance to the measured concentration or density of one or more of the marker components (e.g., endogenous secreted molecule
concentrations or phenotypic profiles) described above. Examples of cytokines that may be added to the culture media include stem cell factors (e.g., KITL), flt3 ligand (FLT3L), and thrombopoietin (THPO), among others.

The use of this device may be useful for improving culture growth of cells, such as human or non-human blood stem and progenitor cells (e.g., including hematopoietic cells).

### Examples

Examples of the disclosed devices and methods, including studies comparing its use to conventional cell culture methods, are described below. These examples are for the purposes of illustration only and are not intended to be limiting. Although certain theories and models are put forth, the disclosure may not be held to any such theories or models and may not be dependent on any such theories or models.

Mathematical simulations may be performed using a model for the cell culture, for example the model described in [4], to explore the impact of culturing cells with a controlled culture media delivery approach. The simulated *in vitro* and *in vivo* functional assay outputs included total nuclear cells (TNC), colony-forming cells (CFC), long term culture-initiating cells (LTCIC) and *Scid* mouse repopulating cells (SRC), which have been shown to be robust assays for the quantification of the expansion of total cells, committed progenitors, primitive progenitors, and stem cells, respectively.

FIG. 2 illustrates the results of example mathematical simulations for controlled culture media delivery, in this example using the model described in [4]. The simulations predicted that controlled culture media delivery would lead to significant increases in all functional assay outputs using an example of the disclosed devices and methods, with outcomes being dependent on the rate of culture media delivery. For example, FIG. 2A shows predicted day 8 functional outputs for total nuclear cells (TNC), colony forming cells (CFC), long term culture-initiating cells (LTCIC) and *Scid* mouse repopulating cells (SRC) at various dilution rates. Additional simulations suggested that these improvements may be a result of decreased concentrations of inhibitory secreted factors in culture in the cell vessel as the culture media in the cell vessel was continuously diluted. For example, FIG. 2B shows simulated day 8 concentrations of hypothetical negative secreted factors (SF1 and SF2) at various dilution rates.

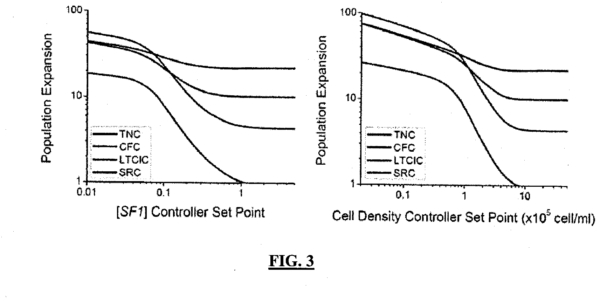

A mathematical model, such as one which specifies certain timing and amount of medium supplementation and selection as in the example described above, may be used to predicatively guide delivery of culture media. FIG. 3 shows outputs from an example computational model for predicting the effects of culture media control on stem and progenitor cell growth. 8-day cultures using an example of the disclosed device were simulated with culture media dilutions rates based on controlling secreted inhibitory protein concentration (left) or cell density (right), over a range of controller set points.

Mathematical models may also be used together with a feedback control strategy. For example, such mathematical models may be used to set predetermined initial media delivery rates and/or set predetermined threshold level(s) for marker component(s).

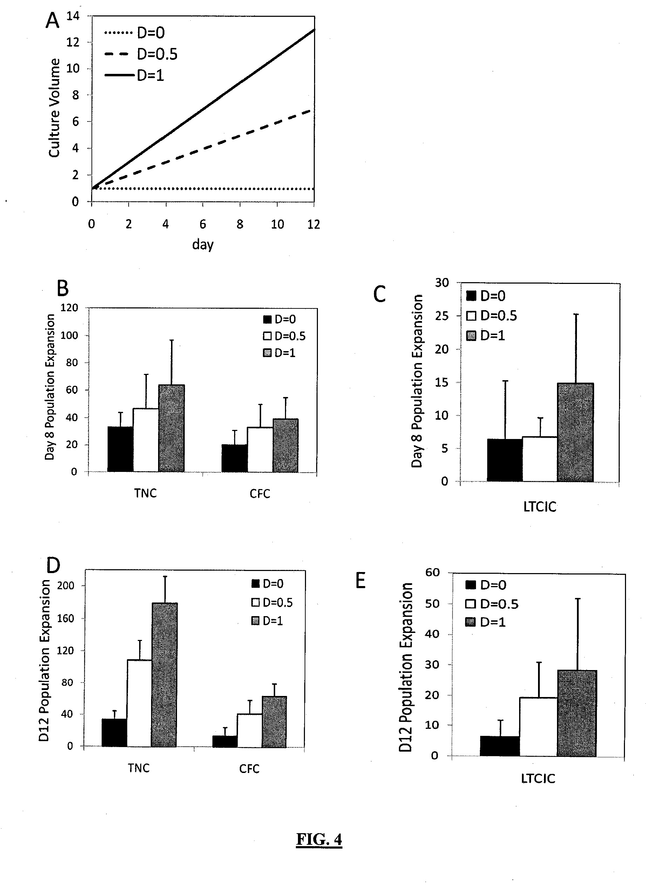

FIG. 4 illustrates an example study comparing functional outputs of cell culture using an example of the disclosed device versus conventional methods. For initial experimental validation, cells were cultured in serum-free media, for example as described in [6]. For controlled culture media delivery, culture media was delivered to the cell vessel semi-continuously or substantially continuously, at a constant delivery rate, again with the dilution rate, D, indicating the fold increase of media that was delivered to the culture vessel each day, as compared to the starting volume. For example, FIG. 4A shows media volume in the cell vessel over time at different constant dilution rates. Thus, for a constant dilution rate of D = 1, the volume of culture media that was added over each 24h period would be equal to the initial culture volume in the cell vessel.

The functional assay outputs achieved from a controlled culture media delivery strategy at two different dilution rates are shown for cells on day 8 and day 12 of culture in FIGS. 4B-C and FIGS. 4D-E, respectively. FIGS. 4B and 4C shows the day 8 expansion data of total nucleated cells (TNC), colony-forming cells (CFC), and long term-culture initiating cells (LTCIC), comparing a fed-batch delivery of dilution rate D=0.5 and D=1, to control cells cultured in a standard constant volume
culture with a media exchange every 4 days (D=0). FIGS. 4D and 4E shows similar data to FIGS. 4B and 4C for day 12 of culture. At both time points, the controlled culture media delivery approach outperformed the cells subject to conventional culture conditions (D=0), based on all assays performed, which appears to agree with predictions by the model in [4].

At day 8, the level of LTCIC expansion obtained from the controlled culture media delivery approach at D=1 was more than double what is seen with the conventional culture (15.0X as compared to 6.5X). Notably, whereas previous UCB culture strategies have seen a decline in primitive progenitor numbers after 8 days, the controlled culture media delivery approach may allow for continued primitive progenitor expansion up to the 12 day time-point. The LTCIC expansion reached 28.6X on day 12 in the controlled culture media delivery (D=1) cultures.

Other controlled culture media delivery rates may be used (e.g., with more complexity), which may more closely mimic the culture dynamics of specific cells and/or factors of interest. In the example below, a variety of variable delivery schemes were compared that each gave the same level of total culture volume in the cell vessel on day 8 but which used different dynamics of dilution.

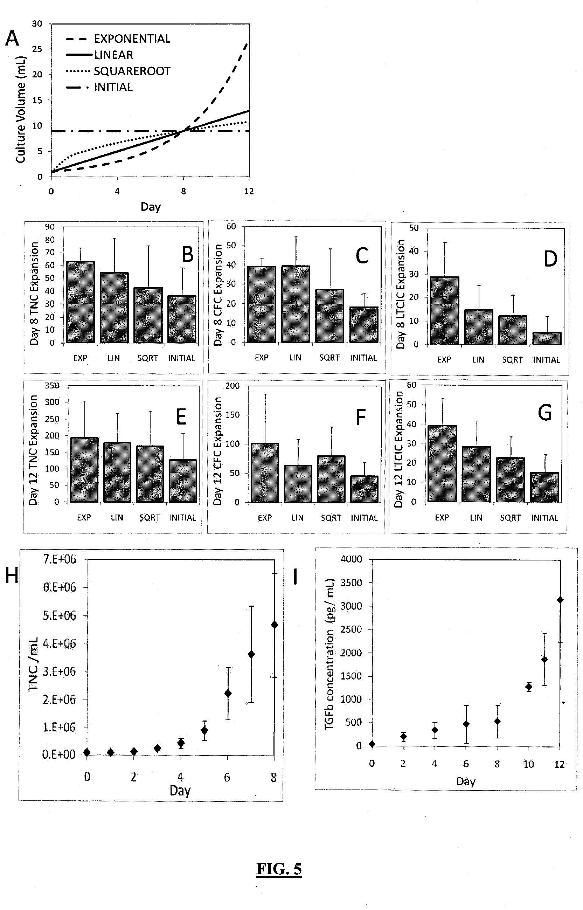

Reference is now made to FIG. 5, illustrating different example dilution rates using an example of the disclosed device, and the results of using the different dilution rates. The different culture media delivery rates are shown in FIG. 5A as the change of culture media volume in the cell vessel over time. The functional assay outputs are shown in FIGS. 5B, 5C, 5D, 5E, 5F and 5G. FIG. 5B shows day 8 total nucleated cell expansions comparing the delivery strategies illustrated in FIG. 5. FIG. 5C shows day 8 colony forming cell expansions. FIG. 5D shows day 8 long term culture-initiating cell expansions. Interestingly, the exponential strategy of culture media delivery seemed to produce the greatest expansions on day 8 compared to the other example delivery strategies, giving rise to population expansion folds of 63X, 39X and 28X for TNC, CFC and LTCIC respectively. FIGS. 5E, 5F and 5G show similar data to FIGS. 5B, 5C and 5D for day 12 of culture. Referring to FIGS. 5H and 5I, respectively showing the time course of total cell expansion in a standard 8 day culture; and the time course of TGFβ secretion in a standard 8 day culture, using an ELISA assay, it appears that both cell growth rate and rate of secretion of critical
factors follow an exponential curve in standard culture conditions. It may be that a corresponding exponential culture media delivery rate most closely tracks one of these critical parameters and thus may provide improved results.

In an example study, the results of controlled culture media delivery were compared to the direct inhibition of negative factors on the cell culture. Previous work has identified several secreted factors that have a negative influence on HSC self-renewal [4]. Of these, TGFβ emerged as a very strongly negative factor, as has also been previously reported [7, 8]. The results of controlled culture media delivery using the disclosed device was compared to results from the addition of a TGFβ inhibitor in order to determine whether simply adding a small molecule inhibitor would produce comparable results.

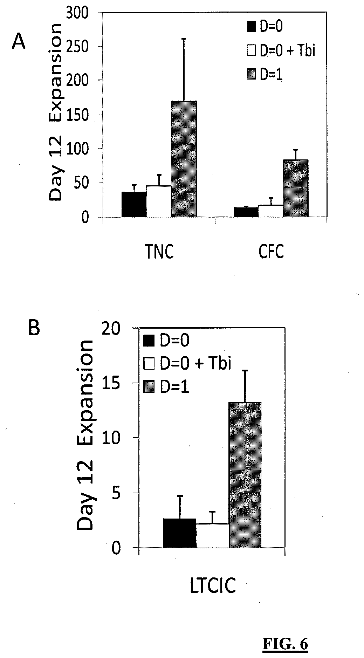

FIG. 6 illustrates a comparison of culture growth using an example of the disclosed devices and methods versus a conventional method with the additional of an inhibitor, in an example study. FIG. 6A and 6B show day 12 population expansions comparing the growth of cells in a controlled culture media delivery strategy of constant dilution rate D=1 to that using a conventional culture method with the addition of 1µM of the TGFβ inhibitor, SB431542 (TBi). As shown in FIGS. 6A and 6B, the controlled culture media delivery approach outperforms the results achieved with the TGFβ inhibitor alone in a conventional method. These results suggest that a more global regulation of the culture system (e.g., by general dilution of the culture media in the cell vessel) may be more effective than the inhibition of a single factor. Moreover, inhibitors may not be available for all negative factors present in culture and a controlled culture media delivery method may relatively easily achieved, such as without the need to test, combine, and/or optimize the use of many different inhibitors.

In some examples, the disclosed devices and methods may control the culture media delivery rate using a dynamic strategy (e.g., using monitoring of one or more marker components and feedback control), which may be based on counteracting the increasing concentration of endogenously produced negative regulators. As the accumulation of many secreted factors follow similar temporal trajectories as cell culture progresses, monitoring and controlling the culture system based on the level of one or more marker components (e.g., a single representative critical factor, such as
TGFβ) may be a feasible strategy to be used. The concentration of the marker component(s), in some examples secreted factor(s), can be measured periodically, continuously or intermittently for example as sampled by a controller device or manually (e.g., using conventional methods, such as an enzyme-linked immunosorbent assay (ELISA)) or using on-line sensors, and the culture media delivery rate (which affects the dilution in the cell vessel) may be adjusted accordingly (e.g., through manual or automatic control of a delivery mechanism) to maintain the concentration of the marker component at, above or below a predetermined threshold.

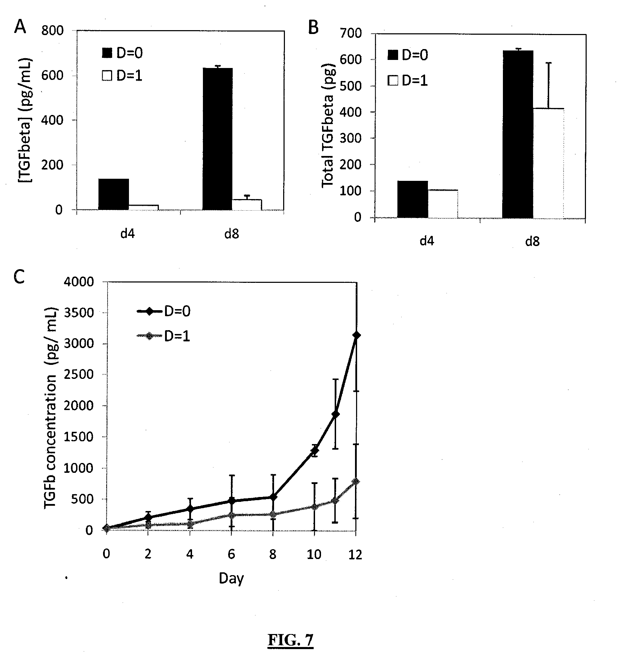

An example of such feedback control is shown in FIG. 7. In this example, control of the culture media delivery rate is based on monitored levels of endogenously secreted factors. FIG. 7A shows total TGFβ (pg) present in cultures using an example of the disclosed device (D=1) and a conventional method (D=0) using ELISA assay. FIG. 7B shows TGFβ concentration (pg/mL) in cultures using an example of the disclosed device (D=1) and a conventional method (D=0), which explicitly takes into account the difference in culture volume. The controlled culture media delivery approach, using an example of the disclosed devices and methods, increases the culture volume, thereby decreasing the concentration of TGFβ and maintaining it at a level where its inhibitory effect on the stem cell population may be reduced. FIG 7C shows a time course of TGFβ concentration (pg/mL) comparing an example of the disclosed device (D=1) and a conventional method (D=0).

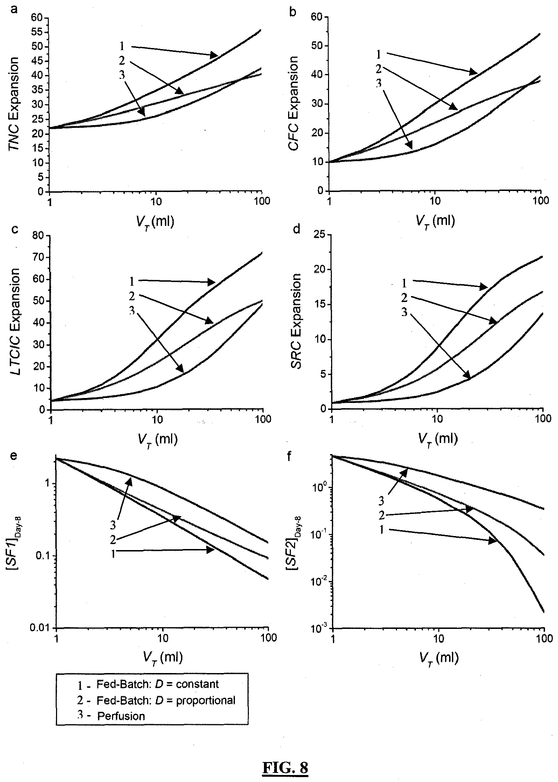

Reference is now made to FIG. 8, showing the results of an example simulation comparing examples of controlled culture media delivery without perfusion to a conventional culture method using perfusion. In the charts shown, "fed-batch" is used to refer to controlled culture media delivery. FIG. 8 shows 8-day fold expansions of TNC (FIG. 8A), CFC (FIG. 8B), LTCIC (FIG. 8C), SRC (FIG. 8D), and final (day-8) media concentrations of theoretical proliferation inhibitor SF1 (FIG. 8E) and self-renewal inhibitor SF2 (FIG. 8F) as functions of media dilution rate, normalized to total volume (V_{T}) of media required. The results suggest that a cell culture method using controlled culture media delivery, such as in the disclosed devices and methods, may provide improved output compared to a conventional culture method using perfusion. The absence of perfusion in the controlled culture
media delivery method may help to avoid the need to remove media from the cell vessel, which may reduce disturbances to the culture.

In some examples, the disclosed devices and methods may allow for the use of perfusion in addition to controlled culture media delivery, for example by providing an outlet in the cell vessel for removing media. The combination of controlled culture media delivery and perfusion (or removal of culture media) may be useful. For example, removal of waste media from the cell vessel may be useful in further removing inhibitors that inhibit cell growth or output, and/or allowing the control of the concentration of inhibitory factors or of inhibitory cell types without substantially increasing the culture volume. Removal of waste media may also be controlled in a manner similar to the control of culture media delivery (e.g., continuous, substantially-continuous or feedback-based).

### Example stimulators

Using controlled culture media delivery, all endogenous factors are diluted at the same rate, including both inhibitors and stimulators. Stimulators may be positive endogenous factors (e.g., certain proteins) that help to promote a desired cell output or cell behavior. In some examples, one or more stimulators may be added and/or reintroduced back into the cell vessel, for example as a soluble factor, to counteract the fact that endogenous positive factors are being diluted. In some examples, in addition to controlled delivery of culture media to the cell vessel, the device may also include controlled delivery of stimulators to the cell vessel.

For example, growth factors or cytokines such as SCF, TPO, FLT-3L, and others, or the role of the transcription factors HOXB4 and the engineered fusion gene between NUP98 and the homeodomain of HOXA10 (NUP98A10HD), provided as soluble membrane-permeable proteins, have been considered as clinically relevant reagents to enhance *in vitro* HSC self-renewal. Other suitable stimulators may include, for example, megakaryocyte-derived stimulatory growth factors (e.g., VEGF, PDGF, EGF and serotonin). Strategies for the delivery of the TAT-HOXB4 and TAT-NUP98A10HD fusion proteins to umbilical cord blood cultures may be developed and carried out using an example of the disclosed devices and methods, for example to achieve continuous or semi-continuous protein delivery, and may be based on a
suitable predictive model, for example to predict dynamic intracellular protein concentrations. It may be that a continuous or substantially continuous delivery approach is useful for unstable proteins, such as TAT-HOXB4, and the delivery of such unstable factors, may be suitable with a culture media dilution strategy as described above.

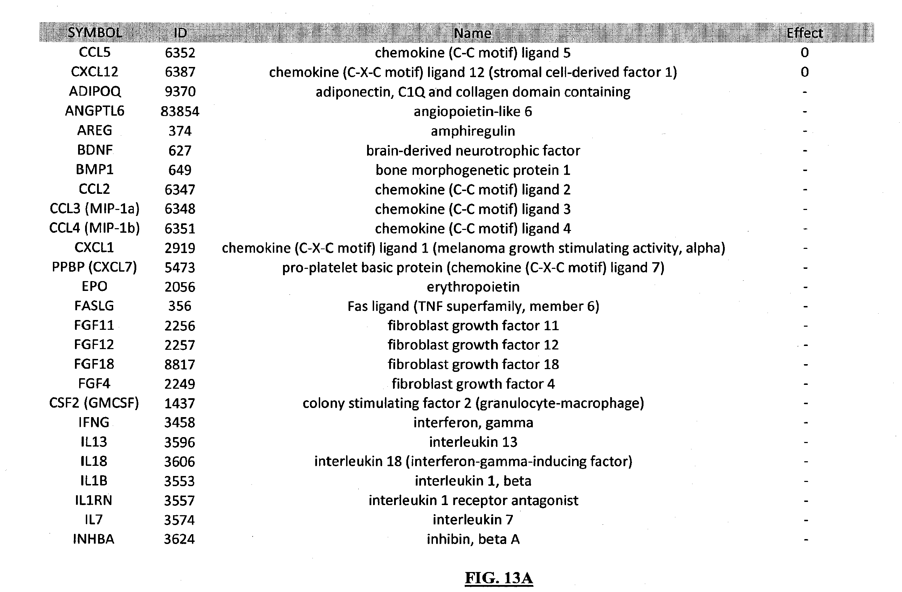

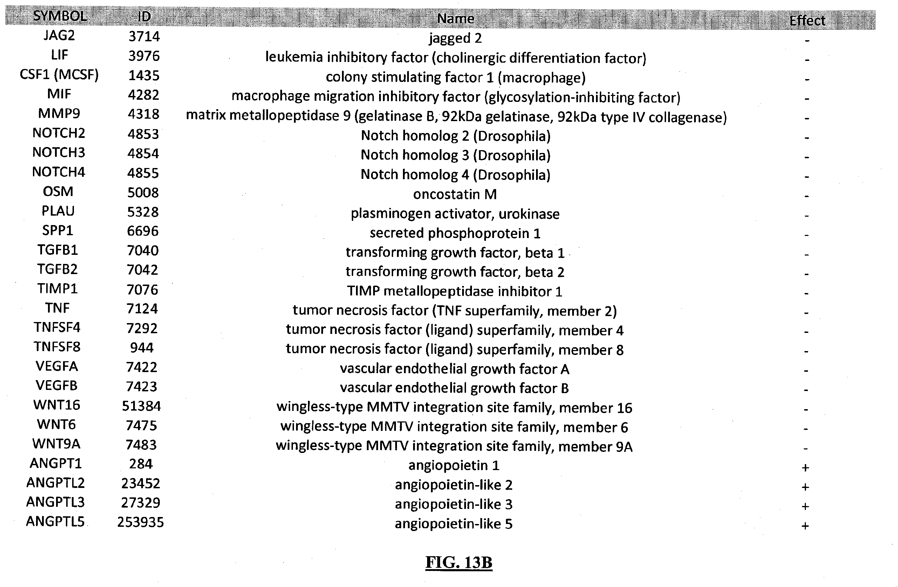

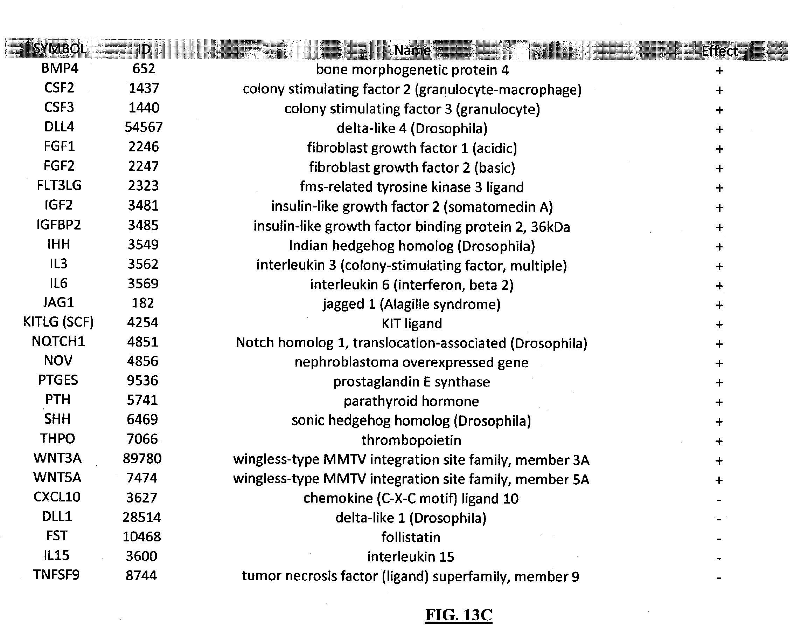

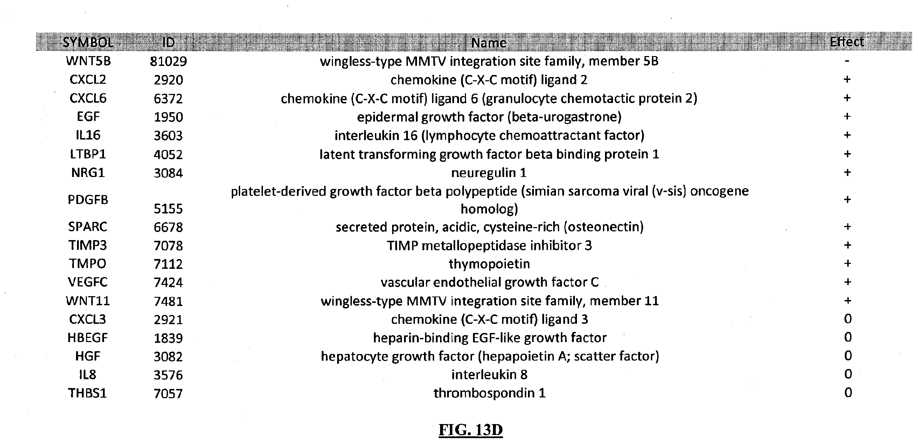

In some example studies, it has been found that an optimized delivery scheme of 1.5nM (from day 0-4) and 6nM (from day 4-8) every 30min, produces stable intracellular levels of TAT-HOXB4, and results in a increase of primitive progenitor cells, as measured by colony counts from bulk long term culture-initiating cell (LTC-IC) assays, of 1.9x greater than the classic, non-optimized TAT-HOXB4 delivery scheme (40nM every 4h) and 3.1x greater than untreated control cells. Other example studies consider HSC self-renewal using the NOD/SCID repopulating cell assay. These example studies may suggest that endogenously produced secreted factors limit HSC output, and that TAT-HOXB4 acts to desensitize the primitive blood progenitor cells to negative feedback regulation by secreted factors. Other suitable stimulators may include, for example, the growth factors SCF, TPO and FLT-3L, as well as EGF, VEGF, PDGF, and other suitable growth factors. Other suitable stimulators may include, for example, those listed in FIGS. 13A-13D, described below.

In some examples, one or more stimulators may be delivered to the cell vessel through the addition of the stimulator(s) directly to the culture media being delivered. The stimulator(s) may be directly added to the culture media in a fixed concentration. In this way, the delivery of stimulator(s) may be indirectly controlled (i.e., through the control of culture media delivery). In some examples, the delivery of one or more stimulators may be directly controlled, for example by using another controlled delivery mechanism (e.g., a controlled valve or pump) for delivering the stimulator(s) to the cell vessel (e.g., through an additional inlet separate from the delivery of culture media). Such direct control of stimulator delivery may be similar to the control of culture media delivery. For example, the delivery of one or more stimulators may be controlled based on a predetermined delivery rate, such as determined by a mathematical model or based on feedback information.

### Mathematical model

An example mathematical model is now described that may be suitable as a basis for controlling culture media delivery. Although an example model is described, this is only for the purpose of illustration and is not intended to be limiting. The present disclosure may not be dependent on the model and its workings.

An example of a suitable mathematical model, for example of hematopoiesis, may integrate findings from various experimental and/or theoretical studies. Such an example model may be implemented as series of ordinary differential equations wherein cell-level kinetic parameters (e.g., proliferation and self-renewal rates) are defined as functions of secreted molecule-mediated inter-cellular networks. By relation to quantitative cellular assays, such an example model may be useful for predictively simulating features of both normal and malignant hematopoiesis, which may be useful for relating internal parameters and microenvironmental variables to measurable cell fate outcomes.

In one example, which may be suitable for blood cell cultures, the mathematical model is a feedback-based cell-cell interaction network model of hematopoiesis. In the example model, the hematopoietic hierarchy can be divided into discrete cellular compartments, wherein compartment transitions are typically coincident with compartment size amplifying cell divisions. Taking advantage of differentiation-state-associated in vitro and in vivo assays, functional readouts have been defined as overlapping series of consecutive compartments. The functional readouts considered are the immuno-deficient *(Non-Obese Diabetic* (*NOD*)/*Scid*) mouse repopulating cell (SRC) assay for quantifying stem cells, the long-term culture-initiating cell (LTC-IC) assay for quantifying primitive progenitors, and the colony forming cell (CFC) assay for quantifying committed progenitors. Hematopoietic cell populations are also broadly classified phenotypically based on their expression (Lin⁺), or lack of expression (Lin⁻) of cell surface antigens associated with differentiated blood cells. Estimates for cell compartment-assay relationships, were undertaken as described in [4].

Gaussian-type functions were used to define the proliferation rate (*uᵢ*) and the self-renewal probability (*fᵢ*) as a function of compartment number (*i*) based on the
internal constants *u_{MAX}*, *n_{MAX}*, *D_{GR}*, and *D_{SR}* (detailed further in [4]). A branching model of hematopoiesis was simulated by lumping differentiated (Lin⁺) cells into 3 functional classes based on their functional feedback interactions with stem and progenitor cells during propagation; populations that secrete inhibitors, populations that secrete stimulators, and populations that secrete molecules with no net effect. Compartment-specific self-renewal and proliferation rates were designated as regulated by the balance of endogenously secreted inhibitors (negative feedback) and stimulators (positive feedback). Based on the above, the resultant example mathematical model includes of 24 state variables [20 cell compartments (*Xᵢ*) and 4 secreted regulatory molecules (*SF1-4*)], and 16 internal parameters, their definitions and theoretically constrained ranges given in [4].

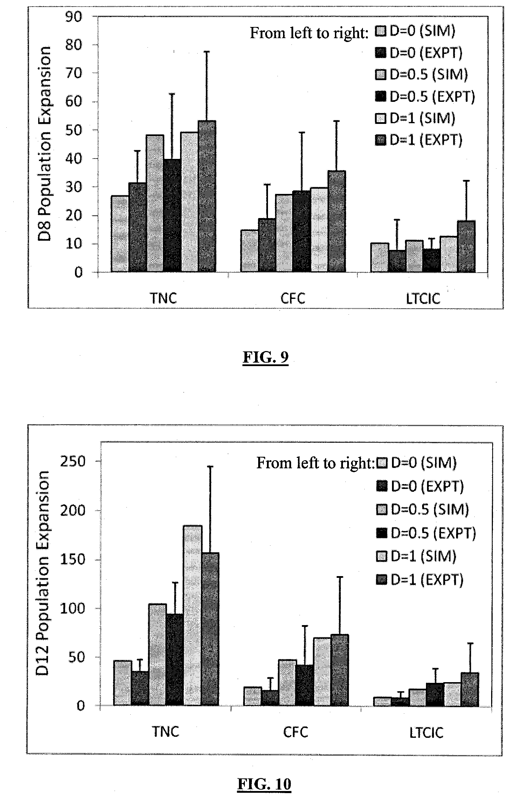

The example mathematical model described may be used to predict the functional culture outputs at different dilution rates, thereby allowing for *in silico* optimization of the culture system, which may be experimentally validated. As shown in FIGS. 9 and 10, the model predictions of outputs resulting from a controlled culture media delivery at fixed dilution rates D=0.5 and D=1 agree with the experimental results at both the 8 day point and 12 day point, suggesting that the model may be useful for the disclosed devices and methods. Additionally, model simulations may be used to investigate the effects of different variable culture media delivery strategies, to help predict the effects of different delivery rates on culture outputs.

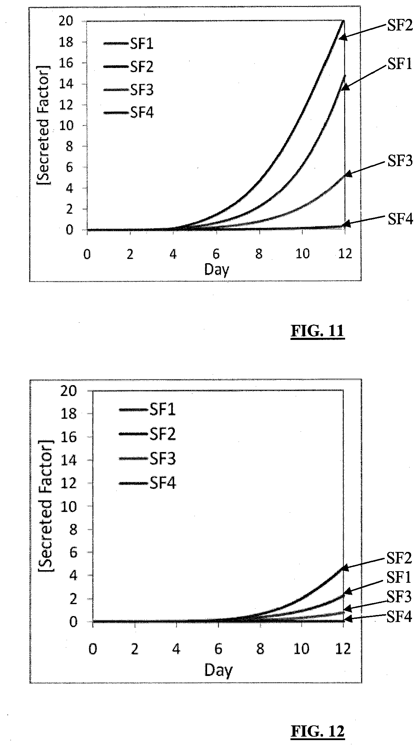

Within the example model, all secreted factors are represented by four categories: proliferation inhibitors (SF1), self-renewal inhibitors (SF2), proliferation stimulators (SF3) and self-renewal stimulators (SF4). Although each individual factor may be secreted at a different rate, the model predicts that the secretion rate of each category of factor will follow an exponential curve, as shown in FIG. 11. When controlled culture media delivery is carried out, the concentrations of all of these factors are decreased, as indicated in FIG. 12, thereby reducing their paracrine impact on the cells in culture.

The controlled culture media delivery approach may involve the continuous or substantially continuous (e.g., periodically or intermittently) dilution of the culture media in the cell vessel, in some examples with fresh serum-free media being supplemented with stimulators to help promote culture output, for example with the
cytokines, stem cell factor (SCF) at 100ng/mL, Flt-3 ligand (FL) at 100ng/mL and thrombopoietin (TPO) at 50ng/mL. By continuously or substantially continuously adding this stimulatory media, while simultaneously diluting all endogenously produced factors, the culture system may be skewed towards a stem-cell supportive environment.

In some examples, in addition to the cytokine supplementation described above, the addition of other factors can be combined with the continuous or substantially continuous controlled culture media delivery. For example, using a continuous or substantially continuous delivery approach for the delivery of unstable TAT-fusion proteins (TAT-HOXB4, TAT-NUP98HOXA10) may be useful, and in some examples the continuous or substantially continuous delivery of these types of labile proteins may be used with controlled culture media delivery.

A large variety of endogenous factors are typically secreted in hematopoietic cell culture. Examples of these factors have been identified from literature as well as through a culture systems-level molecular profiling study and categorized as stimulators or inhibitors of stem cell expansion (for example in [4]) and they are summarized in FIGS. 13A-13D. In these tables, column 2 indicates Entrez gene ID numbers. Under the "Effect" column "+" indicates that the factor has known stimulatory effects, "-" indicates that the factors has known inhibitory effects, and "0" indicates that the factor has no known effect. Where there is a gene alias for the factor, it is provided in parentheses in column 1. A number of these factors have been experimentally validated and several have been categorized more specifically as stimulators or inhibitors of self-renewal or proliferation, which correlate to secreted factor categories, SF1-SF4, in the example mathematical model.

### Example system

An example embodiment of the device for culturing cells is now described. The device may be based on a fed-batch delivery mechanism and may include a control process to modulate the concentration level of certain components (e.g., critical secreted factors) in the cell culture.

In an example embodiment, the cell vessel may be a bag (e.g., a flexible bag made of a plastic such as Teflon), a culture flask or plate (e.g., made of a plastic such
as polystyrene), or a stirred bioreactor vessel, among others. The example cell vessel includes one or more inlets or ports for receiving, for example, culture media. The cell vessel may be relatively small in size (e.g., about 1 mL in volume) or may be larger (e.g., on the order of several litres), depending on the required media volumes used and/or cell population sizes.

Culture media may be delivered to the cell vessel via the inlet(s). In this example, a reservoir of culture media may be kept in a syringe or other container (e.g., in a temperature-controlled environment), and may be connected to the inlet(s) (e.g., via a capillary or tubing) for controlled delivery to the cell vessel. A delivery mechanism delivers the culture media to the cell vessel. In this example, the delivery mechanism may be a pumping system (e.g., a syringe-loaded pump, a peristaltic pump or other suitable pump). The delivery mechanism may be controlled using, for example, a processor. In this example, a software program, such as a Labview-based program, may be executed by a processor and used to control operation of the pumping system, to allow for user-defined control of media delivery.

An example of a suitable bag-based cell vessel system is described in Csaszar et al. [5]. Other variations of such systems may be used.

In this example embodiment, delivery of the culture media may be controlled based on predetermined fixed or variable volume addition, or may be based on more complex delivery profiles based on, for example, model simulation predictions, off-line (e.g., static or not real-time) measurement of certain variable(s) and/or on-line (e.g., dynamic or real-time) measurement of certain variable(s). Delivery of the culture media may be controlled to be, for example, substantially continuously (e.g., at a steady or changing rate), intermittent (e.g., at irregular intervals), periodic (e.g., at regular intervals) or combinations therefore. Delivery of the culture media may be based on, for example, a predetermined schedule or may be dynamic based on monitoring of the system conditions (e.g., concentration of certain factors in the vessel media).

For example, mathematic models (e.g., the model described in Kirouac et al. [4] or variations thereof) may be used to predict or determine delivery profiles for a desired cell culture behaviour, such as expansion of cell (e.g., stem cell) population.
Such models may be based on predetermined and/or monitored information including, for example, information about cell culture conditions (e.g., cell population size) and/or accumulation of certain factors in the cell vessel.

In an example study, based on the model described in Kirouac et al. [4], it was validated that a substantially continuous delivery of culture media where the media is delivered following an exponentially increasing delivery rate, over a 12 day period, resulted in a significant expansion of primitive progenitor stem cell populations. It has also been found that media delivery strategies in which the volume of culture media in the cell vessel is diluted (e.g., in the range of about 5 to about 25 times dilution from the starting volume by the end of the incubation period, such as 12 days) resulted in positive cell population expansion for umbilical cord blood cells.

In some example embodiments, monitoring of marker component(s) in the culture media in the cell vessel may be performed with the aid of one or more sensors. The use of sensor(s) may allow for on-line (e.g., dynamic or real-time) monitoring. For example, Kirouac et al. [10] describes secreted proteins in a cell culture that may be monitored and their concentrations controlled, using the above-described device, for example. The monitoring may be based on a marker component that may be the secreted factors that are to be controlled or may be a surrogate that is indicative of or associated with the secreted factors.

In the example embodiment, off-line (e.g., static or not real-time) monitoring of the marker component(s) may be based on intermittent or periodic sampling of the culture media in the cell vessel (e.g., through the inlet(s) or other ports). Monitoring may involve, for example, performing a concentration quantification assay (e.g., an ELISA assay). For example, the concentration of the factor TGFβ may be monitored using an off-line ELISA assay. In an example study, control of the concentration of TGFβ was found to be useful for promoting expansion of progenitor stem cell populations. Additionally or alternatively, monitoring of the marker component(s) may be performed on-line (e.g., dynamically or real-time). In an example embodiment, on-line monitoring may be performed using one or more sensors, such as a bead-based barcoding sensor, for example as described in Klostranec et al. [9].

In some examples, such as where one or more sensors are used for on-line monitoring of marker component(s), data obtained from monitoring may be transmitted, for example to a processor running software controlling the delivery mechanism, to control the delivery mechanism dynamically or in real-time. This may allow the delivery of fresh culture media to be controlled in order to maintain concentration of marker component(s) (and by extension certain factors of interest) below predetermined threshold concentrations. For example, an example study has found that in some cases maintaining the concentration of secreted TGFβ below a concentration of about 300 pg/mL through dilution of the culture media, such as described above, results in improved cell population expansion as compared to a conventional system. Conventionally, over a culture period of 12 days, TGFβ concentration levels may reach 3000 pg/mL or higher.

While the present disclosure refers to the use of the device and the controlled culture media delivery method for culturing stem cells, the present disclosure may be applicable to other cell types, including, for example, stem cells, progenitor cells, and non-stem cells, for both human and non-human cells. Although certain culture media delivery strategies and rates have been described, other delivery rates may be suitable, including, for example, constant rate, linear rate, exponential rate, sinusoidal rate, step rate, among others. Where the present disclosure refers to continuous or substantially continuous control or delivery of culture media, it should be understood that regular or periodic control or delivery of culture media may also be suitable. For example, within the time frame of a typical culture, which is on the order of days, periodic control or delivery of culture media on the order of hours may be considered to be substantially continuous. Although certain marker components, stimulators and inhibitors have been described, it should be understood that these are for the purpose of illustration only and other marker components, stimulators and/or inhibitors may be considered.

The embodiments of the present disclosure described above are intended to be examples only. Alterations, modifications and variations to the disclosure may be made without departing from the intended scope of the present disclosure. In particular, selected features from one or more of the above-described embodiments may be combined to create alternative embodiments not explicitly described. The
subject matter described herein intends to cover and embrace all suitable changes in technology. All references mentioned are hereby incorporated by reference in their entirety.

### References

1. Douay, L., Experimental culture conditions are critical for ex vivo expansion of hematopoietic cells. J Hematother Stem Cell Res, 2001. 10(3): p. 341-6.
2. Madlambayan, G.J., et al., Controlling culture dynamics for the expansion of hematopoietic stem cells. J Hematother Stem Cell Res, 2001. 10(4): p. 481-92.
3. Robinson, S., et al., Ex vivo expansion of umbilical cord blood. Cytotherapy, 2005. 7(3): p. 243-50.
4. Kirouac, D.C., et al., Cell-cell interaction networks regulate blood stem and progenitor cell fate. Mol Syst Biol, 2009. 5: p. 293.
5. Csaszar, E., et al., An automated system for delivery of an unstable transcription factor to hematopoietic stem cell cultures. Biotechnol Bioeng, 2009. 103(2): p. 402-12.
6. Madlambayan, G.J., et al., Dynamic changes in cellular and microenvironmental composition can be controlled to elicit in vitro human hematopoietic stem cell expansion. Exp Hematol, 2005. 33(10): p. 1229-39.
7. Blank, U., G. Karlsson, and S. Karlsson, Signaling pathways governing stem-cell fate. Blood, 2008. 111(2): p. 492-503.
8. Majka, M., et al., Numerous growth factors, cytokines, and chemokines are secreted by human CD34(+) cells, myeloblasts, erythroblasts, and megakaryoblasts and regulate normal hematopoiesis in an autocrine/paracrine manner. Blood, 2001. 97(10): p. 3075-85.
9. Klostranec, J.M., et al., Convergence of quantum dot barcodes with microfluidics and signal processing for multiplexed high-throughput infectious disease diagnostics. Nano Lett, 2007. 7(9): p. 2812-8.
10. Kirouac, D.C., et al., Dynamic Interaction Networks in Hierarchically Organized Tissue. In Review at Mol Syst Biol, 2010.

## Claims

1. The use of a system for culturing at least one hematopoietic stem cells and/or progenitor cells comprising:
a cell vessel for culturing cells, having an inlet for receiving culture media; and
a delivery mechanism connected to the inlet of the cell vessel for delivering the culture media to the cell vessel, the delivery mechanism being controlled to continuously or intermittently deliver the culture media at a determined culture media delivery rate; and
a controller device configured to:
- receive, continuously or intermittently, signals representing measurement of at least one marker component, wherein the measurement is a concentration or density of the at least one marker component;
- determine the culture media delivery rate for diluting concentration of the at least one marker component in the cell vessel based on the measurement of the at least one marker component; and
- control the delivery mechanism;
wherein the marker component is at least one of: an endogenous secreted factor, wherein the endogenous secreted factor is selected from: ADIPOQ, CCL2, CCL3, CCL4, CCL5, CXCL7, CXCL8, CXCL1O, EGF, PDGF, TGFB1, TGFB2, TNFSF9, and VEGF; and a cell population, wherein the cell population is selected from: CD14, CD15, CD33, CD41, CD235a, CD133, CD34, CD38, CD71 and Rho123 expressing cells.

2. The use of claim 1, wherein the controller device is further configured to:
- receive, continuously or intermittently, signals representing measurement of the number of cells and/or a total number of nucleated cells (TNC) of the cell population; and;
- determine the culture medium delivery rate for diluting the concentration of the at least one marker in the cell vessel based on the measurement of the number of cells and/or the TNC.

3. The use of claim 1 or 2 wherein the information on the concentration of the at least one marker component is based on a sample of cell vessel media.

4. The use of any one of claims 1 to 3 further comprising a sensor for monitoring, continuously or intermittently, the concentration of the at least one marker component.

5. The use of any one of claims 1 to 4 wherein the cell vessel further comprises an outlet for removing waste media.

6. The use of claim 5 further comprising a waste removal mechanism controlled to remove the waste media at a determined waste media removal rate.

7. The use of any one of claims 1 to 6 wherein the culture media delivery rate is determined in order to maintain the measured concentration or density of the at least one marker component below a predetermined threshold value.

8. The use of any one of claims 1 to 7 wherein the at least one marker component is TGFB1.

9. The use of any one of claims 1 to 8 wherein the cell vessel further comprises a second inlet for continuously or intermittently receiving at least one stimulator at a determined stimulator delivery rate.

10. A method of culturing hematopoietic stem cells and/or progenitor cells comprising:
measuring, continuously or intermittently, a concentration or a density of at least one marker component in a cell culture:
calculating, continuously or intermittently, using a culture behavior model, a culture media delivery rate based on the measured concentration or density of the at least one marker component; wherein the marker component is at least one of an endogenous secreted factor, the endogenous secreted factor is selected from: ADIPOQ, CCL2, CCL3, CCL4, CCL5, CXCL7, CXCL8, CXCL1O, EGF, PDGF, TGFB1, TGFB2, TNFSF9 and VEGF; and a cell population, wherein the cell population is selected from: CD14, CD15, CD33, CD41, CD235a, CD133, CD34, CD38, CD71, Rho123 expressing cells;
delivering, continuously or intermittently, culture media to the cell culture at the calculated culture media delivery rate, in order to dilute concentration of the at least one marker component in the cell culture.

11. The method of claim 10, further comprising:
measuring, continuously or intermittently, the number of cells and/or a total number of nucleated cells (TNC) in the cell culture of the cell population; and
calculating, continuously or intermittently, the culture media delivery rate based on the concentration of the at least one marker in the cell vessel based on the measurement of the number of cells and/or the TNC.

12. The method of claim 10 or 11, wherein the culture media delivery rate is determined in order to maintain the measured concentration or density of the at least one marker component below a predetermined threshold value.

13. The method of any one of claims 10 to 12, wherein the at least one marker component is TGFB1.

14. The method of any one of claims 10 to 13 further comprising continuously or intermittently removing waste media from the cell culture at a calculated waste media removal rate.

15. The method of claim 14 wherein the waste media removal rate is calculated based on the measured concentration of the at least one marker component or at least another one marker component.

16. The method of any one of claims 10 to 15 further comprising continuously or intermittently delivering one or more stimulators to the cell culture at a calculated stimulator delivery rate.

17. The method of claim 16 wherein the stimulator delivery rate is calculated based on the measured concentration of the at least one marker component or at least another one marker component.

18. The method of claim 16 or claim 17 wherein the at least one stimulator is at least one of:
- a growth factor, preferably including at least one of: EGF, VEGF, PDGF, SCF, TPO, serotonin and FLT-3L;
- a cytokine, preferably including at least one of: a stem cell factor, a flt3 ligand, and thrombopoietin; and
- a fusion protein, preferably including at least one of: a TAT-HOXB4 fusion protein and a TAT-NUP98A10HD fusion protein.

## Patentansprüche

1. Verwendung eines Systems zum Kultivieren wenigstens einer hämatopoetischen Stammzelle und/oder Progenitorzelle, das umfasst:
ein Zellgefäß zum Kultivieren von Zellen mit einem Einlass zum Aufnehmen von Kulturmedium; und
einen Zufuhrmechanismus, der mit dem Einlass des Zellgefäßes verbunden ist, zum Zuführen des Kulturmediums zu dem Zellgefäß, wobei der Zufuhrmechanismus so geregelt ist, dass das Kulturmedium kontinuierlich oder periodisch mit einer bestimmten Zufuhrrate zugeführt wird; und
eine Regelungsvorrichtung, die ausgelegt ist zum:
- Empfangen, kontinuierlich oder periodisch, von Signalen, die den Messwert für wenigstens eine Markerkomponente darstellen, wobei der Messwert eine Konzentration oder Dichte der wenigstens einen Markerkomponente ist;
- Bestimmen der Zufuhrrate für das Kulturmedium zum Verdünnen der Konzentration der wenigstens einen Markerkomponente in dem Zellgefäß auf Basis des Messwerts für die wenigstens eine Markerkomponente; und
- Regeln des Zufuhrmechanismus;
wobei die Markerkomponente wenigstens eines ist von: einem sezernierten endogenen Faktor, wobei der sezernierte endogene Faktor ausgewählt ist aus: ADIPOQ, CCL2. CCL3, CCL4, CCL5, CXCL7, CXCL8, CXCL10, EGF, PDGF, TGFB1, TGFB2, TNFSF9 und VEGF; und einer Zellpopulation, wobei die Zellpopulation ausgewählt ist aus: CD14, CD15, CD33, CD41, CD235a, CD133, CD34, CD38, CD71 und Rho123-exprimierenden Zellen.

2. Verwendung nach Anspruch 1, wobei die Regelungsvorrichtung weiterhin ausgelegt ist zum:
- Empfangen, kontinuierlich oder periodisch, von Signalen, die den Messwert für die Anzahl von Zellen und/oder eine Gesamtzahl kernhaltiger Zellen (TNC) der Zellpopulation darstellen; und
- Bestimmen der Zufuhrrate für das Kulturmedium zum Verdünnen der Konzentration des wenigstens einen Markers in dem Zellgefäß auf Basis des Messwerts für die Anzahl von Zellen und/oder die TNC.

3. Verwendung nach Anspruch 1 oder 2, wobei die Information über die Konzentration der wenigstens einen Markerkomponente auf einer Probe von Zellgefäßmedium basiert.

4. Verwendung nach einem der Ansprüche 1 bis 3, weiterhin umfassend einen Sensor zum Monitoring, kontinuierlich oder periodisch, der Konzentration der wenigstens einen Markerkomponente.

5. Verwendung nach einem der Ansprüche 1 bis 4, wobei das Zellgefäß weiterhin einen Auslass zum Abführen von Abfallmedium umfasst.

6. Verwendung nach Anspruch 5, weiterhin umfassend einen Mechanismus zur Abfallabführung, der so geregelt ist, dass das Abfallmedium mit einer bestimmten Abführrate für das Abfallmedium abgeführt wird.

7. Verwendung nach einem der Ansprüche 1 bis 6, wobei die Zufuhrrate für das Kulturmedium so bestimmt wird, dass die gemessene Konzentration oder Dichte der wenigstens einen Markerkomponente unter einem vorbestimmten Schwellenwert gehalten wird.

8. Verwendung nach einem der Ansprüche 1 bis 7, wobei die wenigstens eine Markerkomponente TGFB1 ist.

9. Verwendung nach einem der Ansprüche 1 bis 8, wobei das Zellgefäß weiterhin einen zweiten Einlass umfasst, um wenigstens einen Stimulator kontinuierlich oder periodisch mit einer bestimmten Zufuhrrate für den Stimulator aufzunehmen.

10. Verfahren zum Kultivieren hämatopoetischer Stammzellen und/oder Progenitorzellen, umfassend:
Messen, kontinuierlich oder periodisch, einer Konzentration oder einer Dichte wenigstens einer Markerkomponente in einer Zellkultur;
Berechnen, kontinuierlich oder periodisch, unter Verwendung eines Kulturverhaltensmodells, einer Zufuhrrate für das Kulturmedium auf Basis der gemessenen Konzentration oder Dichte der wenigstens einen Markerkomponente; wobei die Markerkomponente wenigstens eines ist von: einem sezernierten endogenen Faktor, wobei der sezernierte endogene Faktor ausgewählt ist aus: ADIPOQ, CCL2. CCL3, CCL4, CCL5, CXCL7, CXCL8, CXCL10, EGF, PDGF, TGFB1, TGFB2, TNFSF9 und VEGF; und einer Zellpopulation, wobei die Zellpopulation ausgewählt ist aus: CD14, CD15, CD33, CD41, CD235a, CD133, CD34, CD38, CD71 und Rho123-exprimierenden Zellen; Zuführen, kontinuierlich oder periodisch, von Kulturmedium zu der Zellkultur mit einer berechneten Zufuhrrate für das Kulturmedium, um die Konzentration der wenigstens einen Markerkomponente in der Zellkultur zu verdünnen.

11. Verfahren nach Anspruch 10, weiterhin umfassend:
Messen, kontinuierlich oder periodisch, der Anzahl von Zellen und/oder einer Gesamtzahl kernhaltiger Zellen (TNC) in der Zellkultur der Zellpopulation; und
Berechnen, kontinuierlich oder periodisch, der Zufuhrrate des Kulturmediums auf Basis der Konzentration des wenigstens einen Markers in dem Zellgefäß auf Basis des Messwerts für die Anzahl von Zellen und/oder die TNC.

12. Verfahren nach Anspruch 10 oder 11, wobei die Zufuhrrate für das Kulturmedium so bestimmt wird, dass die gemessene Konzentration oder Dichte der wenigstens einen Markerkomponente unter einem vorbestimmten Schwellenwert gehalten wird.

13. Verfahren nach einem der Ansprüche 10 bis 12, wobei die wenigstens eine Markerkomponente TGFB1 ist.

14. Verfahren nach einem der Ansprüche 10 bis 13, weiterhin umfassend kontinuierliches oder periodisches Abführen von Abfallmedium aus der Zellkultur mit einer berechneten Abführrate für das Abfallmedium.

15. Verfahren nach Anspruch 14, wobei die Abführrate für das Abfallmedium auf Basis der gemessenen Konzentration der wenigstens einen Markerkomponente oder wenigstens noch einer Markerkomponente berechnet wird.

16. Verfahren nach einem der Ansprüche 10 bis 15, weiterhin umfassend kontinuierliches oder periodisches Zuführen von ein oder mehr Stimulatoren zu der Zellkultur mit einer berechneten Zufuhrrate für den Stimulator.

17. Verfahren nach Anspruch 16, wobei die Zufuhrrate für den Stimulator auf Basis der gemessenen Konzentration der wenigstens einen Markerkomponente oder wenigstens noch einer Markerkomponente berechnet wird.

18. Verfahren nach Anspruch 16 oder Anspruch 17, wobei der wenigstens eine Stimulator wenigstens eines ist von:
- einem Wachstumsfaktor, bevorzugt beinhaltend wenigstens eines von: EGF, VEGF, PDGF, SCF, TPO, Serotonin und FLT-3L;
- einem Zytokin, bevorzugt beinhaltend wenigstens eines von: einem Stammzellfaktor, einem flt3-Liganden und Thrombopoietin; und
- einem Fusionsprotein, bevorzugt beinhaltend wenigstens eines von: einem TAT-HOXB4-Fusionsprotein und einem TAT-NUP98A10HD-Fusionsprotein.

## Revendications

1. Utilisation d'un système pour cultiver au moins une cellule souche hématopoïétique et/ou cellule progénitrice comprenant :
un récipient à cellules pour cultiver des cellules, ayant une entrée pour recevoir un milieu de culture ; et
un mécanisme de fourniture relié à l'entrée du récipient à cellules pour fournir le milieu de culture au récipient à cellules, le mécanisme de fourniture étant commandé pour fournir de manière continue ou intermittente le milieu de culture à un débit de fourniture de milieu de culture déterminé ; et
un dispositif de commande configuré pour :
- recevoir, de manière continue ou intermittente, des signaux représentant la mesure d'au moins un composant marqueur, où la mesure est une concentration ou une densité du au moins un composant marqueur ;
- déterminer le débit de fourniture de milieu de culture pour diluer la concentration du au moins un composant marqueur dans le récipient à cellules sur la base de la mesure du au moins un composant marqueur ; et
- commander le mécanisme de fourniture ;
où le composant marqueur est au moins l'un de : un facteur sécrété endogène, où le facteur sécrété endogène est choisi parmi : ADIPOQ, CCL2, CCL3, CCL4, CCL5, CXCL7, CXCL8, CXCL10, EGF, PDGF, TGFB1, TGFB2, TNFSF9 et VEGF ; et une population de cellules, où la population de cellules est choisie parmi: les cellules exprimant CD14, CD15, CD33, CD41, CD235a, CD133, CD34, CD38, CD71 et Rho123.

2. Utilisation selon la revendication 1, où le dispositif de commande est configuré en outre pour :
- recevoir, de manière continue ou intermittente, des signaux représentant la mesure du nombre de cellules et/ou d'un nombre total de cellules nucléées (TNC) de la population de cellules ; et
- déterminer le débit de fourniture de milieu de culture pour diluer la concentration du au moins un marqueur dans le récipient à cellules sur la base de la mesure du nombre de cellules et/ou du TNC.

3. Utilisation selon la revendication 1 ou 2 où l'information sur la concentration du au moins un composant marqueur est basée sur un échantillon de milieu du récipient à cellules.

4. Utilisation selon l'une quelconque des revendications 1 à 3 comprenant en outre un capteur pour suivre, de manière continue ou intermittente, la concentration du au moins un composant marqueur.

5. Utilisation selon l'une quelconque des revendications 1 à 4 où le récipient à cellules comprend en outre une sortie pour retirer le milieu constituant un déchet.

6. Utilisation selon la revendication 5 comprenant en outre un mécanisme de retrait de déchet commandé pour retirer le milieu constituant un déchet à un débit de retrait de milieu constituant un déchet déterminé.

7. Utilisation selon l'une quelconque des revendications 1 à 6 où le débit de fourniture de milieu de culture est déterminé pour maintenir la concentration ou densité mesurée du au moins un composant marqueur en dessous d'une valeur seuil prédéterminée.

8. Utilisation selon l'une quelconque des revendications 1 à 7 où le au moins un composant marqueur est TGFB1.

9. Utilisation selon l'une quelconque des revendications 1 à 8 où le récipient à cellules comprend en outre une seconde entrée pour recevoir de manière continue ou intermittente au moins un stimulant à un débit de fourniture de stimulant déterminé.

10. Procédé de culture de cellules souches hématopoïétiques et/ou de cellules progénitrices comprenant :
la mesure, de manière continue ou intermittente, d'une concentration ou d'une densité d'au moins un composant marqueur dans une culture de cellules ;
le calcul, de manière continue ou intermittente, au moyen d'un modèle de comportement de culture, d'un débit de fourniture de milieu de culture sur la base de la concentration ou densité mesurée du au moins un composant marqueur, où le composant marqueur est au moins l'un d'un facteur sécrété endogène, le facteur sécrété endogène est choisi parmi : ADIPOQ, CCL2, CCL3, CCL4, CCL5, CXCL7, CXCL8, CXCL10, EGF, PDGF, TGFB1, TGFB2, TNFSF9 et VEGF ; et d'une population de cellules, où la population de cellules est choisie parmi : les cellules exprimant CD14, CD15, CD33, CD41, CD235a, CD133, CD34, CD38, CD71 et Rho123,
la fourniture, de manière continue ou intermittente, de milieu de culture à la culture de cellules au débit de fourniture de milieu de culture calculé, pour diluer la concentration du au moins un composant marqueur dans la culture de cellules.

11. Procédé selon la revendication 10, comprenant en outre :
la mesure, de manière continue ou intermittente, du nombre de cellules et/ou d'un nombre total de cellules nucléées (TNC) dans la culture de cellules de la population de cellules ; et
le calcul, de manière continue ou intermittente, du débit de fourniture de milieu de culture sur la base de la concentration du au moins un marqueur dans le récipient à cellules sur la base de la mesure du nombre de cellules et/ou du TNC.

12. Procédé selon la revendication 10 ou 11, où le débit de fourniture de milieu de culture est déterminé pour maintenir la concentration ou densité mesurée du au moins un composant marqueur en dessous d'une valeur seuil prédéterminée.

13. Procédé selon la revendication 10 ou 12, où le au moins un composant marqueur est TGBF1.

14. Procédé selon l'une quelconque des revendications 10 à 13 comprenant en outre le retrait de manière continue ou intermittente de milieu constituant un déchet de la culture de cellules à un débit de retrait de milieu constituant un déchet calculé.

15. Procédé selon la revendication 14 où le débit de retrait de milieu constituant un déchet est calculé sur la base de la concentration mesurée du au moins un composant marqueur ou d'au moins un autre composant marqueur.

16. Procédé selon l'une quelconque des revendications 10 à 15 comprenant en outre la fourniture de manière continue ou intermittente d'un ou plusieurs stimulants à la culture de cellules à un débit de fourniture de stimulant calculé.

17. Procédé selon la revendication 16 où le débit de fourniture de stimulant est calculé sur la base de la concentration mesurée du au moins un composant marqueur ou d'au moins un autre composant marqueur.

18. Procédé selon la revendication 16 ou la revendication 17 où le au moins un stimulant est au moins l'un de :
- un facteur de croissance, de préférence incluant au moins l'un de : EGF, VEGF, PDGF, SCF, TPO, la sérotonine et FLT-3L ;
- une cytokine, incluant de préférence au moins l'un de : un facteur de cellules souches, un ligand de flt3 et la thrombopoïétine ; et
- une protéine de fusion, incluant de préférence au moins l'une de : une protéine de fusion TAT-HOXB4 et une protéine de fusion TAT-NUP98A10HD.
